# EUROPEAN PATENT APPLICATION

(11) **EP 2 433 942 A1**
(43) Date of publication of application: **28.03.2012**
(21) Application number: 10777372.3
(22) Date of filing: 20.05.2010
(51) Int. Cl.: C07D 495/04, C07D 235/00, C07D 333/00

(54) **METHOD FOR PREPARING D-BIOTIN**

(30) Priority: 21.05.2009 CN 200910098678
(71) Applicant: Zhejiang Medicine Co., Ltd., Shaoxing, Zhejiang 312500 (CN)
(72) Inventor: PAN, Yajin, Shaoxing Zhejiang 312500 (CN); PI, Shiqing, Shaoxing Zhejiang 312500 (CN); DING, Wenzhen, Shaoxing Zhejiang 312500 (CN); GU, Lixin, Shaoxing Zhejiang 312500 (CN); WEI, Angfeng, Shaoxing Zhejiang 312500 (CN); HE, Yimin, Shaoxing Zhejiang 312500 (CN)
(74) Representative: Schmid, Klaus Michael Johannes
(86) International application number: PCT/CN2010/072977
(87) International publication number: WO 2010/133169

(57) **Abstract**

An Improved Preparation Method for D-Biotin

The invention discloses an improved preparation method for D-Biotin. If the composite method takes the diesteas the raw material, the finial biotin will be generated with impurities. The features of the invention refers to the improved preparation method for D-Biotin includes: firstly, (3aS,4S,6aR)-1,3-ωωω -butyl)-1*H*-thiaphene[3,4-d]-2,4(1*H)*-ketone is got after the methane tricarboxylic acid trialkyl ester and (3aR, 8aS, 8bS)-1,3-2-[3,4-d]thiophene [1,2-a]sulfuryl halide have the condensation reaction in the alkaline environment and then D-Biotin can be got after the reaction process of hydrolysis, decarbaxylation and closed loop. The invention succeeds in greatly improving the biotin quality from the original basis and simultaneously avoids the generation of impurities and the occurrence of side reaction.

## Description

### Technical Field

The invention involves in the biotin preparation field, especially the improved preparation method for D-Biotin.

### Background Art

D-Biotin, also known as Vitamin H, is mainly used in the fields of medicine and sanitation, nutrition enhancer, feed additive, cosmetics and drinks, etc. The molecular structural formula of D-Biofin is stated as follows:

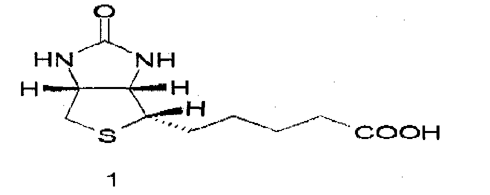

Since the Roche Corporation firstly succeed in the industrial synthesis of D-Biotin in 1949, foreign counties have continued to carry out a great quantity of research on it, and up until now, there have been several synthesis methods for D-Biotin. But the sternbach synthesis method which takes the thiolactone as the key intermediate is still the acknowledged synthetic route with industrialization significance.

Sternbach route takes the thiolactone 2 as the initial material with optical activity. And after several reactions, key intermediate (3aR, 8aS, 8bS)-1,3-dibenzyl -2-oxo-10H- iminazole[3,4-d] thiophene[1,2-a] sulfuryl bromide 3 can be got And then in the alkaline environment, the compound 4 can be got after the bromide 3 and propandioic acid diethyl ester have the condensation reaction. Finally after the a series of reaction process of open circle with acid treatment, hydrolyzation, decarboxylation, benzyl off and closed loop, Biotin 1 can be got. And the relevant synthetic route is listed as follows:

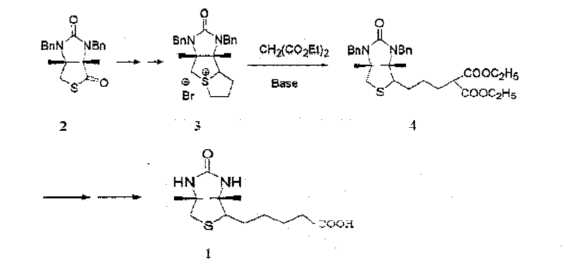

Whereof, in the condensation reaction process of bromide 3 propandioic acid diester, for there are two active hydrogen in the propandioic acid diester, the condensation compound gained after the reaction of two active hydrogen and bromide 3 will continue to react with bromide 3 in the Ikaline environment and then the impurity 5 can be got. After the further reaction process of hydrolysis, and closed loop for condensation compound 4, finally the Biotin 1 can be got but with impurity 6. Though the impurity can be reduced by the increase of propandioic acid diester or by the method of recrystallization, the impurity can not be eliminated in the final biotin. It is the defects of the process by using the propandioic acid diester which could generate the above impurity. The generation mechanism and chemical reaction equation is listed as follows:

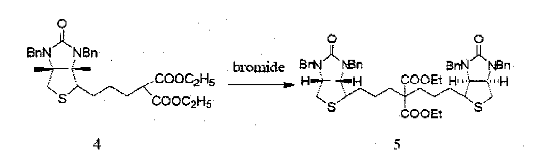

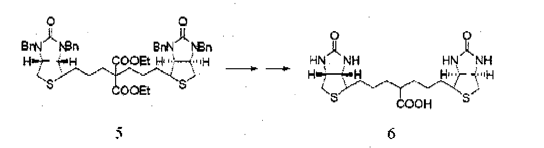

### Summary of invention

The purpose of the invention is to overcome the above defects in the synthetic route and provide an improved preparation method of D-Biotin. The synthetic route improves preparation method of D-Biotin in chemical mechanism further to avoid the generation of impurities.

Therefore the technical program of the invention is stated as follows: the improved preparation method for D-Biotin includes: firstly, (3aS,45,6aR)-1,3-dibenzyl-4-(ω,ω,ω-3-alkoxycarbonyl bytyl)-4H-1*H-*thiophene[3,4-d]iminazole-2,4(1*H)*-ketone is got after the methane tricarboxylic acid trialkyl ester and (3aR, 8aS, 8bS)-1,3-dibenzyl -2-oxo-10H-iminazole [3,4-d] thiophene [1,2-a] sulfuryl halide have the condensation reaction in the alkaline environment and then D-Biotin can be got after the reaction process of hydrolysis, decarboxylation and closed loop.

The invention replaces the propandioic acid diester with methane 3-carboxylic ester. For the methyne structure of methane 3-carboxylic ester has only one active hydrogen and when it has condensation reaction with the intermediate-halide in the alkaline environment, only one production can be generated but not the impurity 5. After further reaction process of hydrolysis, decarboxylation and closed-loap, the final generated Biotin 1 can be got without impurity 6. The invention improves the biotin quality from the original basis and the utilization ratio of the intermediate-halide without the occurrence of side reaction. The specific reaction equation is listed as follows:

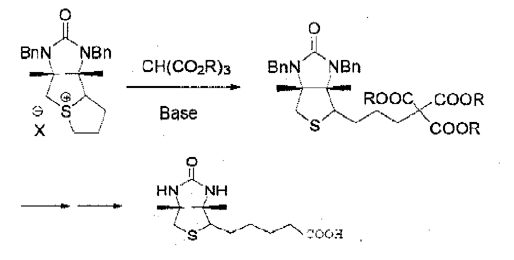

For the invention, the chemical structural formula of the methane tricarboxylic acid trialkyl ester (key raw material is CH(COOR)3, whereof R refers to the alkyl containing 1-5 carbon atom and the R of tricarboxylic ester refers to same or different alkyl. And usually R refers to methyl, ethyl or propyl.

According to the above improved preparation method, the alkali used in the condensation reaction refers to inorganic base or organic base, the mentioned inorganic base refers to sodium hydride, potassium hydride or sodium metal; the mentioned organic base refers to sodium methoxide, sodium ethoxide, sodium tart-butoxide, potassium methoxide, potassium ethoxide or potassium tert-butoxide.

Comparing to the original method of malonic ester, the invention has the following advantages: the quality of the biotin has been greatly improved without impurities; the side reaction has been avoided and the utilization ratio of the intermediate-halide has been greatly improved.

The following embodiments are used for further explaining the invention, but the stated parameters in the embodiments have no restrictions on the invention.

### Specific embodiments

Comparison embodiment

A. Synthesis of the (3aS,45,6aR)-1,3-dibenzyl-4-(ω,ω-2-ethoxyearbonyl-butyl)-4H-1*H*-thiophene[3,4-d]iminazole-2,4(1*H)* -ketone (condensation compound 4)

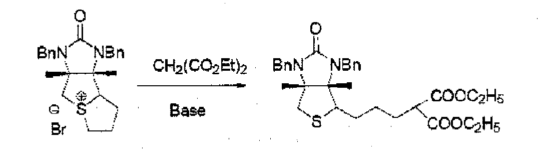

Put 300 ml methylbenzene and 60% sodium hydride 6.0g(0.15 mol) into the 500 ml four-neck flask with reflux condenser pipe, dropping pipette, stirrer and thermometric instrument which is protected by dry nitrogen; and then add 48g (0.3 mol) propandioic acid diethyl ester into the flask at the temperature of below 80°C and protected for 2h by heat preservation; add 33.3g (0.075 mol) bromine sulfonium salts into the flask and then raise the temperature and control the temperature of the entire flask at 80°C for a 15h reaction; cool It to the normal temperature and use 5% sulfuric acid to adjust the pH to pH=3; separate the organic layer and extract the water layer by 100 ml methylbenzene for two times; use 40 ml 5% sodium bicarbonate water solution wash the organic layer for two times; dry the oil layer by anhydrous sodium sulfate, filtrate and reduce pressure to recycle faint yellow fluid, finally get the target object-37g diester dibenzyl biotin (94.1% of the theoretical value), and the HPLC measured content is 94.5% with 3.5% impurity 5 (hereinafter to be referred as dicarboxylic ester).

B. Synthesis of D-Biatin

Put 37g diester dibenzyl biotin and 800 g 48% hydrobromic acid into the 2000 ml three-neck flask, stir, keep reflux and heat preservation at 125-126 °C for 8h; follow the reaction by thin layered method (developing solvent: methylbenzene- glacial acetic acid - methanol =10:10:3(v/v/v)); recycle and eliminate the hydrobromic acid by adding water (3× 100 ml) for three times; alkalize the solution to pH 8-9 by 10% NaOH and cool it at the temperature below 20°C. Weigh 16 g solid phosgene (BTC) and dissolve it with 150 ml methylbenzene; dropwise add the above solution into the water and control the pH of the water layer to be pH 8-9 with alkali; after completing adding process, stir the solution for one hour and use 10% hydrochloric acid adjust the PH to be faintly acid; separate the oil layer and continue to add water into the water layer; finally pure Biotin 1 can be got by activated carbon recrystallization and the HPLC measured content is 96.5°C with 1.2% impurity 6 (hereinafter to be referred as dicarboxylic acid).

Embodiment 1

A. Synthesis of (3aS,45,6aR)-1,3-dibenzyl-4-(ω,ω,ω-3-ethoxycarbonyl-butyl)-4H-1*H*-thiophene[3,4-d]iminazole-2,4(1*H)* - ketone

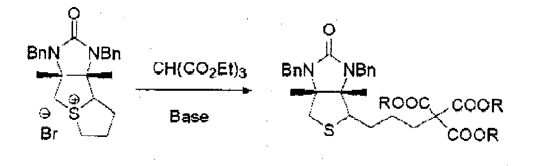

Put 300 ml methylbenzene and 60% sodium hydride 3.3g (0.0825 mol) into the 500 ml four-neck flask with reflux condenser pipe, dropping pipette, stirrer and thermometric instrument which is protected by dry nitrogen; and then add 19.3g (0.083 mol) methane tricarboxylic acid triethyl ester into the flask at the temperature of below 80°C and protected for 2h by heat preservation; add 33.3g (0.075 mol) bromine sulfonium salts into the flask and then raise the temperature and control the temperature of the entire flask at 80°C for a 15h reaction; cool it to the normal temperature and use 5% sulfuric acid to adjust the pH to pH=3; separate the organic layer and extract the water layer by 100 ml methylbenzene for two times; use 40 ml 5% sodium bicarbonate water solution wash the organic layer for two times; dry the oil layer by anhydrous sodium sulfate, filtrate and reduce pressure to recycle faint yellow fluid, finally get the target object-2.5g tri-ester dibenzyl biotin (95.5% of the theoretical value), and the HPLC measured content is 97.8% with no impurity 5 (hereinafter to be referred as dicarboxylic ester).

B. Synthesis of D-Biotin

Put 2.5g diester dibenzyl biotin 4 and 800 g 48% hydrobromic acid into the 2000 ml three-neck flask, stir, keep reflux and heat preservation at 125-126 °C for 8h; follow the reaction by thin layered method (developing solvent: methylbenzene-glacial acetic acid - methanol =10:10:3(v/v/v)); recycle and eliminate the hydrobromic acid by adding water (3×100 ml) for three times; alkalize the solution to pH 8-9 by 10% NaOH and cool it at the temperature below 20°C. Weigh 16 g solid phosgene (BTC) and dissolve it with 150 ml methylbenzene; dropwise add the above solution into the water and control the pH of the water layer to be pH 8-9 with alkali; after completing adding process, stir the solution for one hour and use 10% hydrochloric acid adjust the PH to be faintly acid; separate the oil layer and continue to add water into the water layer; finally pure Biotin 1 can be got by activated carbon recrystallization and the HPLC measured content is 98.7% with no impurity 6 (hereinafter to be referred as dicarboxylic acid).

Embodiment 2

A. Synthesis of (3aS,45,BaR)-1,3-dibenzyl-4-(ω,ω,ω,-3-methoxycarbonyl butyl)-4H-1*H*-hiophene[3,4-d]immazole-2,4(1*H)* - ketone

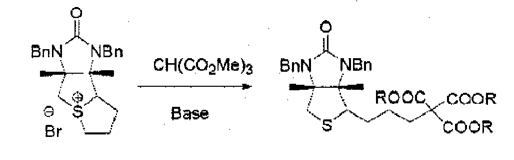

Put 300 ml methylbenzene and 60% sodium hydride 3.3g (0.0825 mol) into the 500 ml four-neck flask with reflux condenser pipe, dropping pipette, stirrer and thermometric instrument which is protected by dry nitrogen; and then add 15.8g(0.083 mol) methane tricarboxylic acid triethyl ester into the flask at the temperature of below 80°C and protected for 2h by heat preservation; add 33.3g (0.075 mol) bromine sulfonium salts into the flask and then raise the temperature and control the temperature of the entire flask at 80°C for a 15h reaction; cool it to the normal temperature and use 5% sulfuric acid to adjust the pH to pH=3; separate the organic layer and extract the water layer by 100 ml methylbenzene for two times; use 40 ml 5% sodium bicarbonate water solution wash the organic layer for two times; dry the oil layer by anhydrous sodium sulfate, filtrate and reduce pressure to recycle faint yellow fluid, finally get the target object-0.2g tri-ssterdibanzyl biotin (95.3% of the theoretical value), and the HPLC measured content is 98.0% with no impurity 5 (hereinafter to be referred as dicarboxylic ester).

B. Synthesis of D-Biotin

Put 0.2g diester dibenzyl biotin 4 and 800 g 48% hydrobromic acid into the 2000 ml three-neck flask, stir, keep reflux and heat preservation at 125-126 °C for 8h; follow the reaction by thin layered method (developing solvent: methylbenzene- glacial acetic acid - methanol =10:10:3(v/v/v)); recycle and eliminate the hydrobromic acid by adding water (3×100 ml) for three times; alkalize the solution to pH 8-9 by 10% NaOH and cool it at the temperature below 20°C. Weigh 16 g solid phosgene (BTC) and dissolve it with 150 ml methylbenzene; dropwise add the above solution into the water and control the pH of the water layer to be pH 8-9 with alkali; after completing adding process, stir the solution for one hour and use 10% hydrochloric acid adjust the PH to be faintly acid; separate the oil layer and continue to add water into the water layer; finally pure Biotin 1 can be got by activated carbon recrystallization and the HPLC measured content is 98.6% with no impurity 6 (hereinafter to be referred as dicarboxylic acid).

Embodiment 3

A. Synthesis of (3aS,4S,6aR)-1,3-dibenzyl-4-(ω,ω,ω-methoxycarbonyl buty)- 4H-1*H*-thiophene[3,4-d]iminazole-2,4(1*H)*- ketone

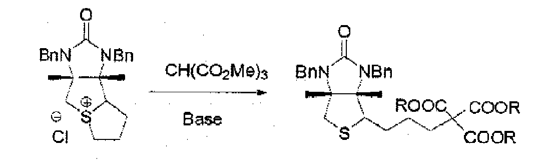

Put 300 ml methylbenzene and 60% sodium hydride 3.3g (0.0825 mol) into the 500 ml four-neck flask with reflux condenser pipe, dropping pipette, stirrer and thermometric instrument which is protected by dry nitrogen; and then add 15.8g (0.085 mol) methane tricarboxylic acid triethyl ester into the flask at the temperature of below 80°C and protected for 2h by heat preservation; add 33.3g (0.075 mol) bromine sulfonium salts into the flask and then raise the temperature and control the temperature of the entire flask at 70°C for a 10h reaction; cool it to the normal temperature and use 5% sulfuric acid to adjust the pH to pH=3; separate the organic layer and extract the water layer by 100 ml methylbenzene for two times; use 40 ml 5% sodium bicarbonate water solution wash the organic layer for two times; dry the oil layer by anhydrous sodium sulfate, filtrate and reduce pressure to recycle faint yellow fluid, finally get the target object-1.0g tri-ester dibenzyl biotin (96.6% of the theoretical value), and the HPLC measured content is 98.4% with no impurity 5 (hereinafter to be referred as dicarboxylic ester).

B. Synthesis of D-Biotin

Put 1.0g diester dibenzyl biotin 4 and 800 g 48% hydrobromic acid into the 2000 ml three-neck flask, stir, Keep reflux and heat preservation at 125-126 °C for 8h; follow the reaction by thin layered method (developing solvent: methylbenzene-glacial acetic acid - methanol =10:10:3(v/v/v)); recycle and eliminate the hydrobromic acid by adding water (3×100 ml) for three times; alkalize the solution to pH 8-9 by 10% NaOH and cool it at the temperature below 20°C. Weigh 16 g solid phosgene (BTC) and dissolve it with 150 ml methylbenzene; dropwise add the above solution into the water and control the pH of the water layer to be pH 8-9 with alkali; after completing adding process, stir the solution for one hour and use 10% hydrochloric acid adjust the PH to be faintly acid; separate the oil layer and continue to add water into the water layer, finally pure Biotin 1 can be got by activated carbon recrystallization and the HPLC measured content is 98.6% with no impurity 6 (hereinafter to be referred as dicarboxylic acid).

Embodiment 4

A. Synthesis of (3aS,45,6aR)-1,3-dibenzyl-4-(ω,ω,ω-3-carbopentyioxy buty)-4H-1*H*-thiophene[3,4-d]iminazole-2,4(1*H)* - ketone

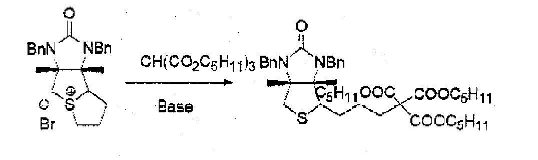

Dissolve 0.2g sodium metal in the namyl alcohol and mix 20.2g (0.087 mol) methane tricarboxylic acid triethyl ester into the above solution, then reflux for 2 hours and reduce the pressure to evaporate the solvent; add 200 ml methylbenzene and 200 ml water, separate the layer, dry the organic layer and reduce pressure to recycle the methylbenzene; methane tricarboxylic acid triamyl ester can be got and dissolve it with 50 ml dry methylbenzene. Put 200 ml methylbenzene and 60% sodium hydride 3.3g (0.0825 mol) into the 500 ml four-neck flask with reflux condenser pipe, dropping pipette, stirrer and thermometric instrument which is protected by dry nitrogen; and then add methylbenzene solution with the above methane tricarboxylic acid triethyl ester into the flask at the temperature of below 80°C and protected for 2h by heat preservation; add 33.3g (0.075 mol) bromine sulfonium salts into the flask and then raise the temperature and control the temperature of the entire flask at 80°C for a 15h reaction; cool it to the normal temperature and use 5% sulfuric acid to adjust the pH to pH=3; separate the organic layer and extract the water layer by 100 ml methylbenzene for two times; use 40 ml 5% sodium bicarbonate water solution wash the organic layer for two times; dry the oil layer by anhydrous sodium sulfate, filtrate and reduce pressure to recycle faint yellow fluid, finally get the target object-4.6g tri-amyl-ester dibenzyl biotin (93.1% of the theoretical value), and the HPLC measured content is 95.8% with no impurity 5 (hereinafter to be referred as dicarboxylic ester).

B. Synthesis of D-Biotin

Put 4.5g diester dibenzyl biotin 4 and 800 g 48% hydrobromic acid into the 2000 ml three-neck flask, stir, keep reflux and heat preservation at 125-126 °C for 8h; follow the reaction by thin layered method (developing solvent: methylbenzene- glacial acetic acid - methanol =10:10:3(v/v/v)); recycle and eliminate the hydrobromic acid by adding water (3×100 ml) for three times; alkalize the solution to pH 8-9 by 10% NaOH and cool it at the temperature below 20°C. Weigh 16 g solid phosgene (BTC) and dissolve it with 150 ml methylbenzene; dropwise add the above solution into the water and control the pH of the water layer to be pH 8-9 with alkali; after completing adding process, stir the solution for one hour and use 10% hydrochloric acid adjust the PH to be faintly acid; separate the oil layer and continue to add water into the water layer; finally pure Biotin 1 can be got by activated carbon recrystallization and the HPLC measured content is 98.7% with no impurity 6 (hereinafter to be referred as dicarboxylic acid).

In conclusion, the above are the relatively good embodiments of the invention, which have no restrictions on the technical program of the invention. All the simple modification, changes and embellishment of the above embodiments according to the material contents of the invention shall be within the protection scope of the invention.

## Claims

1. An improved preparation method for D-Biotin includes: firstly, (3aS,4S,6aR)-1,3-dibenzyl-4-(ω,ω,ω-3-alkoxycarbonyl-bytyl)-4H-1*H-*thiophene[3,4-d]iminazcle-2,4(1*H)*-ketone is got after the methane tricarboxylic acid trialkyl ester and (3aR, 8aS, 8bS)-1,3-dibenzyl -2-oxo-10H-iminazole [3,4-d] thiophene [1,2-a] sulfuryl halide have the condensation reaction in the alkaline environment and then D-Biotin can be got after the reaction process of hydrolysis, decarboxylation and closed loop.

2. The improved preparation method according to Claim 1, the feature of the invention is that the chemical structural formula of the methane tricarboxylic acid trialkyl ester (raw material) is CH(COOR)3, whereof R refers to the alkyl containing 1-5 carbon atom and the R of tricarboxylic ester refers to same or different alkyl.

3. The improved preparation method according to Claim 2, the feature of the invention is that the R of the chemical structural formula of the methane tricarboxylic acid trialkyl ester (raw material) refers to methyl.

4. The improved preparation method according to Claim 2, the feature of the invention is that the R of the methane tricarboxylic acid trialkyl ester (raw material) refers to ethyl.

5. The improved preparation method according to Claim 1, the feature of the invention is that the alkali used in the condensation reaction refers to inorganic base or organic base.

6. The improved preparation method according to Claim 5, the feature of the invention is that the inorganic base used in the condensation reaction refers to sodium hydride, potassium hydride or sodium metal.

7. The improved preparation method according to Claim 5, the feature of the invention is that the organic base used in the condensation reaction refers to sodium methoxide, sodium ethoxide, sodium tert-butoxide, potassium methoxide, potassium ethoxide or potassium tert-butoxide.
